# EUROPEAN PATENT APPLICATION

(11) **EP 4 717 163 A1**
(43) Date of publication of application: **01.04.2026**
(21) Application number: 24203414.8
(22) Date of filing: 27.09.2024
(51) Int. Cl.: A61B 5/11, A61B 5/00

(54) **METHOD AND DEVICE FOR ASSESSING A WALKING PATTERN OF A PERSON**

(71) Applicant: Technische Universität Hamburg, 21073 Hamburg (DE)
(72) Inventor: LANGE, Victor Machado Carneiro, Hamburg (DE); KERN, Thorsten Alexander, Hamburg (DE)
(74) Representative: RGTH

(57) **Abstract**

A method (10) for assessing a walking pattern of a person is suggested, which comprises measuring (13) a roll, a pitch and a yaw of a walking movement of the person as measurement data by means of at least one sensor (101) placed on a pelvis of the person, determining (15) the first derivates of the measurement data as derivation data, and evaluating (18) the measurement data and the derivation data for assessing (17) the walking pattern.

## Description

The present invention relates to a method as well as a device for assessing a walking pattern of a person.

Methods and devices for determining and assessing a walking pattern of a person are generally known from prior art. For example, it is known that a walking steadiness can be determined with devices such as for example a smartphone or a smartwatch. However, the results are not precise enough to provide a comprehensive insight into a walking pattern, for example to monitor exercise improvements beyond a simple increase in speed or frequency, or even complex disorders within single steps.

Therefore, it is an object of the present invention to provide a method as well as a device for assessing a walking pattern of a person which is efficient and precise to provide an actual benefit for the person with regard to diagnosis and/or treatment of walking pattern deviation and/or disorders. Especially, the walking pattern shall be assessed such that a professional can use this information to help or assist the person.

This objective is solved by a method for assessing a walking pattern of a person which comprises measuring a roll, a pitch and at least a yaw of a walking movement of a person as measurement data by means of at least one sensor placed on a pelvis of the person. Further, the method comprises determining the first derivatives of the measurement data as derivation data. In other words, the first derivatives of the measured roll, pitch and yaw are each determined. These form the angular velocities around the specific axis. Further, the second derivates of the yaw, roll and pitch each can be determined and be included in the derivation data. The second derivatives can be understood as angular accelerations.

Further the method comprises evaluating the roll, the pitch and the yaw as measurement data as well as the first derivatives for assessing, or in other words analyzing, the walking pattern.

Since the at least one sensor is placed on the pelvis of the person, the measurement data is very precise when it comes to the walking pattern of the person. The walking pattern can also be referred to as the gait. Since both legs are connected to the hips, the pelvis with the legs can be considered as a parallel mechanism while no muscles are controlling the pelvis rotation directly, but its position depends on the legs as well as the back and core muscles. Thus, measurement data from a sensor which is placed on the pelvis directly can provide essentially improved data regarding accuracy.

The roll can be understood as the pelvic tilt, while the yaw can be understood as the pelvic rotation and the pitch can be understood as the pelvic obliquity. In other words, the pitch, the roll and the yaw are rotations around three different axes of the pelvis of the person. In particular, there are three axis which can be defined as a coordinate system of the pelvis wherein the rotation around the first axis is concerned as the roll. The rotation around the second axis is regarded as the pitch and the rotation around the third axis is considered as the yaw. The first axis is defined as where the transverse and the frontal plane coincide. The second axis is where the transverse and the sagittal plane coincide. The third axis is defined where the sagittal and the frontal plane coincide.

A rotational movement according to the tilt can rotate the pelvis to the anterior or posterior side in a sagittal plane. A rotational movement according to the pitch can move the pelvis up (hike) or down (drop) at one side in the frontal plane. A rotational movement according to the yaw can move the pelvis backward or forward in a transverse plane.

By means of the measurement data as well as the derivation data a more detailed inside and to the dynamics of the movement is possible.

In particular, the measurement data is continuously measured for a period of time, for example for at least one minute, further preferred for at least five minutes, mostly preferred for at least ten minutes, such that a consistency of the data is assured.

In particular, before the roll, the pitch and the yaw are measured, the method can include an additional step of an activity detection based on the measurement data. By evaluating the measurement data an activity of the person such as walking can be identified, such as walking up the stairs, walking, running, sitting. The assessment of the measurement data can begin once a walking activity is recognized.

Before the first derivatives are determined the measurement data can be preprocessed. Furthermore, the data can be extrapolated to a specific frequency, e.g. to 100 Hertz. This facilitates the following assessment of the data.

The measurement data can further include linear accelerations, preferably in three different axis, from which the first and the second derivatives can be determined. Thus, the derivation data can also include the first and/or the second derivatives of the linear accelerations. Thus, preferably the method utilizes angular data, in other words the roll, the pitch and the yaw, as well as linear data about the movement.

In a further preferred embodiment, the method includes a determination of phases of the walking pattern and possibly a subdivision of the measurement data and the derivation data according to the phases of the walking pattern. In other words, a walking pattern can be subdivided or segmented into different phases which are characteristic for a walking pattern. The walking pattern in other words the gait can be subdivided into two phases, namely the stance phase and the swing phase. The cycle begins with one foot touching the ground and ends with the same foot touching the ground again. The period of the walking pattern is from the heel strike through to the toe-off. The interval between sequential initial floor contact by the same limb is called the stride. The stance phase can be subdivided by the initial contact (heel strike), the loading response (foot flat), the mid stance, the terminal stance (heel-off) and the pre-swing (toe-off). While the swing phase can be subdivided into the initial swing, the mid swing and the late swing. Furthermore, there can be even more subdivisions, such as the moment of maximal knee extension.

Furthermore, the method can comprise determining an asymmetry of the walking pattern regarding a left and a right side of the person. In other words, the asymmetry regards a difference regarding the left and the right side. With regard to the phases, a double stance can be determined as well as the right and the left step or in other words stance in the walking pattern. The determination of the asymmetry can include a time analysis and/or frequency analysis and/or time frequency analysis of the measurement data and/or of the derivation data for determining walking pattern features. In particular, the method comprising determining an average in the measurement data and/or derivation data and subtracting it from the data before the features are extracted. This helps to increase the focus on small details by extracting the average.

The walking pattern features can be determined regarding each determined phase of the walking pattern. For example, in the scope of the time analysis different peaks of the measurement data, for example the timing of the peaks and/or the number of the peaks and/or the distance between the peaks can be determined. Furthermore, zero-line crossing events can be detected. Zero-line crossing events means that the pelvis is in line with the respective axis. During a walking cycle, there are positive and negative values (peaks) for the yaw and the pitch such that the measurement data crosses the zero-line regularly. If the individual is healthy when the double support ends (this means the time where both feet are one the ground), the pelvis obliquity should have crossed the line to generate space for the swing. A delay for example could indicate a disorder in the walking pattern.

In particular, the method is computer implemented. In a further preferred embodiment a neural network, in particular a long short-term memory (LSTM) neural network can be employed. The neural network can in particular assist in determining the phases of the walking pattern and/or determining an asymmetry and/or a walking disorder and/or walking deviation.

Further, regarding the time analysis it can be determined whether the measurement data and/or the derivation data exceeds predefined thresholds. Further, the duration of the phases of the walking pattern can be determined as features. In detail, a mean, a variance, an RMS (route means square) and/or a kurtosis can be determined.

In the scope of the time frequency analysis a Fourier synchrosqueezed transform and/or a Wavelet synchrosqueezed transform or any other time-frequency analysis method can be used to extract respective features regarding how the frequencies change over time. For example, it can be determined which frequencies occur related to the time and how much time can be assigned to each frequency. For example, the side lobes of the signals retrieved by at least one of the above transform or analysis methods can be analyzed. By analyzing side lobes, an asymmetry could be seen. Variation in frequencies are easier and more reliably to be seen in time frequency analysis than in other analysis methods.

Regarding the frequency analysis, the frequencies and their change can be observed. For example, in the scope of the frequency analysis, a spectral centroid, a spectral bandwidth, a spectral Flatness and/or a power spectral density could be determined. In this way, the method can detect and analyze shift and frequency components which can occur due to factors such as tiredness, lack of activity, pain or walking pattern adaption for pain mitigation. Further long-term frequency shifts can be detected providing insight into long term gait adaptions or health conditions.

For all of the above-named analysis methods, the root mean square (RMS) of each signal of the measurement data and/or the derivation data, the maximal amplitudes and/or the slopes and/or the number and/or location of local maxima and/or minima can be considered as features.

The determination of the asymmetry comprises a comparison of the measurement data and/or the derivation data and/or walking pattern features to previous data of the same person and/or to a predefined optimal walking pattern. For example, the above-mentioned data and/or features can be compared to a recorded ideal of the person. In addition, or alternatively, the data and/or the features can be compared to a pattern which the person wants to change such that an improvement can be monitored. It can also be compared to an average of the previous steps (of the same side or a different side) such that an improvement can be detected very closely, and it can also be compared to one previous step of the person.

The method can further comprise a comparison of the measurement data and/or the derivation data and/or walking pattern features to predefined data regarding walking pattern disorders, wherein based on the comparison the method comprises providing an indication of a walking pattern disorder and/or walking deviation and possibly its severity. The last term is used for a constant deviation which is not yet considered a disorder. In other words, walking pattern disorders can be predefined. Due to the comparison of the retrieved measurement data and/or the determined derivation data and/or the extracted walking pattern features possible walking pattern disorders could be detected. In particular, the walking pattern disorders with the greatest resemblance regarding the beforementioned data and/or walking pattern features can be indicated. This can help health professionals to diagnose a walking pattern disorder.

Furthermore, the method comprises providing a feedback regarding the walking pattern to the person, in particular a live, in other words real time, feedback. This feedback can for example be provided by vibration signals. In addition, or alternatively, the feedback could be provided visually, for example by means of an application which can for example be installed on a mobile device of the person. In such an application, measurement data and/or features of the walking pattern can be represented, e.g. visually. The feedback shall encourage the person to change its walking pattern.

Furthermore, the feedback can comprise a traffic light system explaining in different visual grades, like a traffic light, a deviation to an optimal walking pattern.

Regarding vibration signals, the method can comprise providing vibration signals at different locations of the pelvis of the person. Furthermore, the vibration signals itself can differ. The vibration signals can vary in intensity and/or duration and/or location and/or frequency. Each signal can have their own meaning indicating to the person different conclusions about the actual walking pattern.

Furthermore, based on the above, a posture of the person can be assessed. The method can also provide feedback regarding the posture as described above. Therefore, the method can relate to measuring the above-described data not only during a walking movement, but also during standing and/or sitting.

Thus, the method is configured to provide very detailed feedback to the person such that the person can change its walking pattern immediately. This helps to monitor training, rehabilitation as well as preventive measures. In other words, the method can track and provide feedback on these aspects facilitating the improvement and maintenance of healthy gait pattern.

Apart from detecting the activity, the person can indicate the activity, such as e.g. stretching. The method is also able to inform about a lack of activities or a variability of activities. It can alert the person about insufficient exercise or inconsistent activity patterns which may impact health. In addition, the quality of short-term activities such as for example stretching can be evaluated from the above data. Therefore, the method can provide a feedback on activity such as exercise and/or stretching, to the person directly, informing the person of the impact on the walking pattern which on one side motivates the person and on the other side makes sure that the person conducts his exercise consistently and correctly.

The method can be configured to conduct a trend analysis on the extracted walking pattern features over a predetermined period of time which offers insights into long term changes in the walking pattern.

Additionally to the gait analysis, the method can include a way to ensure the correct sensor placement by the user. In particular, the method comprises determining a correct position and/or correct pressure of the sensor on the pelvis of the person by measuring and evaluating the vibration signals. Thus, vibration signals can be generated and at the same time measured by the at least one sensor, especially by at least one accelerometer of the sensor, providing feedback to the person on whether the sensor are placed correctly and with sufficient pressure on the pelvis. In detail. The sensor should be placed on a lower part of the back. If the sensor position is not correct, e.g. due to an insufficient tension of the belt, data retrieved at different measurement times are not comparable.

In particular, the at least one sensor is provided on a belt which can be placed around the pelvis of the person. The pressure can relate to a tension of the belt on the pelvis wherein the belt's tension is adjustable, e.g. its length can be adjustable. For example, if the belt is to lose, the sensor does not apply sufficient pressure on the pelvis such that the measurement data is insufficiently accurate. By providing the vibration signals and at the same measuring and evaluating these, a correct position and/or correct pressure can be determined. If this is not the case, feedback to the person can be provided such that an incorrect position and/or incorrect pressure can be corrected. Furthermore, the method can make conclusions about the BMI (body mass index) of the person, since more weight (and thus more tissue) will lead to more damping. In detail, it can extract the BMI of a person based on the determined damping of the vibration signals.

Furthermore, the method can comprise the step of predefining the feedback for individual persons, in other words the persons can customize the feedback such as intensity and/or duration and/or frequency and/or location of the vibration patterns. This customization is important for accommodating individual differences in sensory perception and ensuring comfort during use. For example, it there is more tissue due to weight, there will automatically be more damping of the signal. This will influence the haptic experience of the user. Therefore, the predefinition is optimal to ensure a consistent feedback.

The method is configured to automatically adjust the intensity of the feedback and specially the intensity of the vibration signals depending on the activity of the person. If the person is active, for example the person is running, the intensity can be increased such that it is detected despite of the activity in which the person is typically less sensitive.

In a further aspect the invention relates to a device for assessing a walking pattern of a person, wherein the device includes at least one sensor placed on a pelvis of the person for measuring the pitch, the roll and a yaw of a walking movement of the person as measurement data. Furthermore, the device includes at least one evaluation unit for determining the first and preferably the second derivatives of the measurement data and for evaluating the walking pattern.

In particular, the sensor includes an inertial measurement unit (IMU) which can detect the angles. The IMU can further include at least one accelerometer, at least one gyroscope and at least one magnetometer. Furthermore, the IMU can include one accelerometer for each axis. In particular, the method included only one IMU and no other sensor. Several sensors placed at different parts of the person or for example different parts of the pelvis are not necessary.

The device can further include a feedback actuator to provide a live, in other words real time, feedback, for example a vibration signal, regarding the walking pattern to the person. This actuator can be a vibrotactile actuator. The device can include several actuators placed on different sites on the belt, such that they can apply pressure and/or generate signals at different parts of the pelvis.

In particular, the device includes the belt with which can be placed on the pelvis on the person in a variable way which a variable length such that a pressure on the pelvis can be adjusted depending on the circumference of the person. Based on the adjustable tension the belt is customizable for individual users, wherein the adjustability further ensures that the feedback is consistently and effectively transmitted regardless of the person's body type or clothing.

The invention is designed to provide cues, such for example regarding exercises or changes in walking patterns and/or posture to help persons correct their walking pattern or posture in real-time. Overall, the invention ensures that the feedback is not only responsive and accurate but also adaptable to the needs and preferences of the individual person and enhances the overall effectiveness by providing immediate feedback.

It is shown in only schematic representation:
- Fig. 1:: a method for assessing a walking pattern of a person;
- Fig. 2:: a method for assessing a walking pattern of a person;
- Fig. 3:: a method for assessing a walking pattern of a person;
- Fig. 4:: at least partly a method 10 for assessing a walking pattern of a person;
- Fig. 5:: a part of a method for assessing a walking pattern of a person;
- Fig. 6:: measurement data of the yaw over time;
- Fig. 7:: a device for assessing a walking pattern of a person; and
- Fig. 8:: the roll, yaw and pitch at the pelvis of a person.

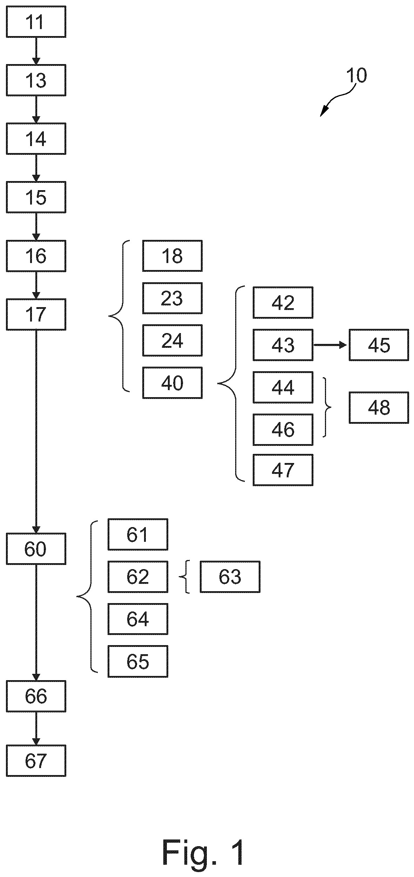

Figure 1 shows a method 10 for assessing a walking pattern of a person.

At first, there can be an activity detection 11 to ensure that the person is active, especially walking. A roll, a pitch and a yaw of a walking movement of a person can be measured 13 as measurement data. Furthermore, linear accelerations can be measured as measurement data 14. The first derivatives of the measurement data can be determined 15 and the second derivatives can be determined 16 as well.

As a next step, the walking pattern can be assessed 17. In particular, this step can include evaluating 18 the measurement data and the derivation data for assessing the walking pattern. Phases of the walking pattern can be determined 23 and the measurement data and/or derivation data can be subdivided 24 according to the phases.

An asymmetry of the walking pattern can be determined 40. This can include a time analysis 42, a frequency analysis 43 and/or a time frequency analysis 44. Based on the resulting walking pattern features 45 an asymmetry can be determined. There can further be different comparisons, such as a comparison 46 to previous data or a comparison 47 to predefined data regarding walking pattern disorders. In this way, also walking disorders or walking deviations can be determined and indicated 48.

As another step, a feedback can be provided 60. This can include a live feedback 61. For example, a vibration signal can be generated 62. Especially, different signals can be provided 63 regarding the location and/or duration and/or frequency and/or intensity of the signal. Furthermore, a visual representation 64 can be provided and/or a traffic light system can be provided 65. In addition, a correct position and/or correct pressure of the sensor on the pelvis can be determined 66 by measuring and evaluating the vibration signals. Furthermore, walking pattern improvements, e.g. due to training or physical exercise, can be tracked 67.

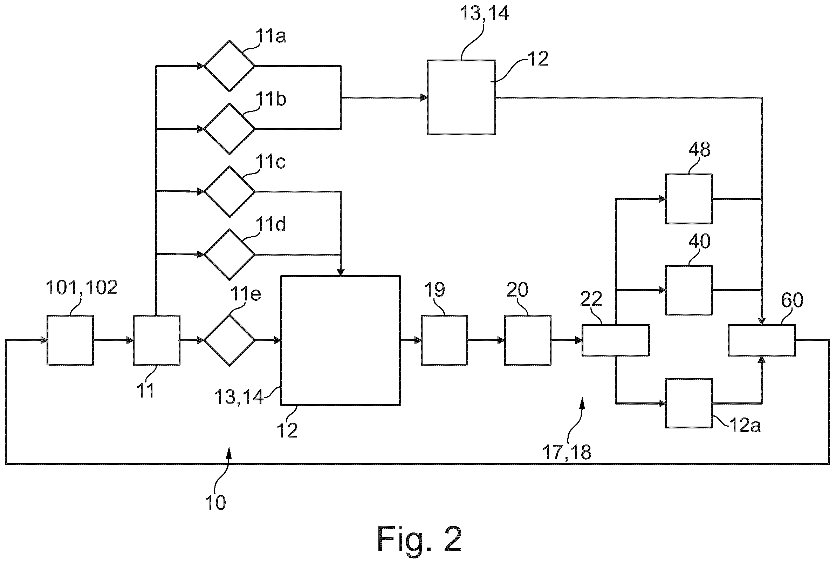

Figure 2 shows a method 10 for assessing a walking pattern of a person. Figure 2 can be based on figure 1 but shows more steps in some regard than figure 1. From the left-hand side, a sensor 101, namely an inertial measurement unit 102, is placed on the pelvis of the person.

As a first step, the method 10 can include an activity detection 11. Different activity types 11a to 11e can be recognized. 11a can stand for sitting, while 11b can stand for standing, 11c can stand for walking up the stairs, while 11d can stand for running. 11e can stand for walking. If either walking up the stairs 11c, running 11d or walking 11e is recognized a preprocessing 12 of the measurement data can take place. The roll, the pitch, the yaw can be measured 13 as well as the linear accelerations can be measured 14. These data can then be preprocessed 12. Also, the measurement data when sitting 11a or standing 11b can be determined and processed 12a.

The data regarding the walking activity can be assessed 17. In other words, the measurement data and possibly the derivation data can be evaluated 18. Double support 19 and left and right step can be determined, and the phases of the walking pattern can be determined 20 as well. For each step, preprocessing 22 can take place. An asymmetry of the walking pattern can be determined 40. Furthermore, a walking disorder and/or walking deviation regarding the walking pattern can be determined and indicated 48. Further processing 12a can take place as well parallelly to the asymmetry determination 40. A feedback 60 can be provided regarding the walking pattern to the person.

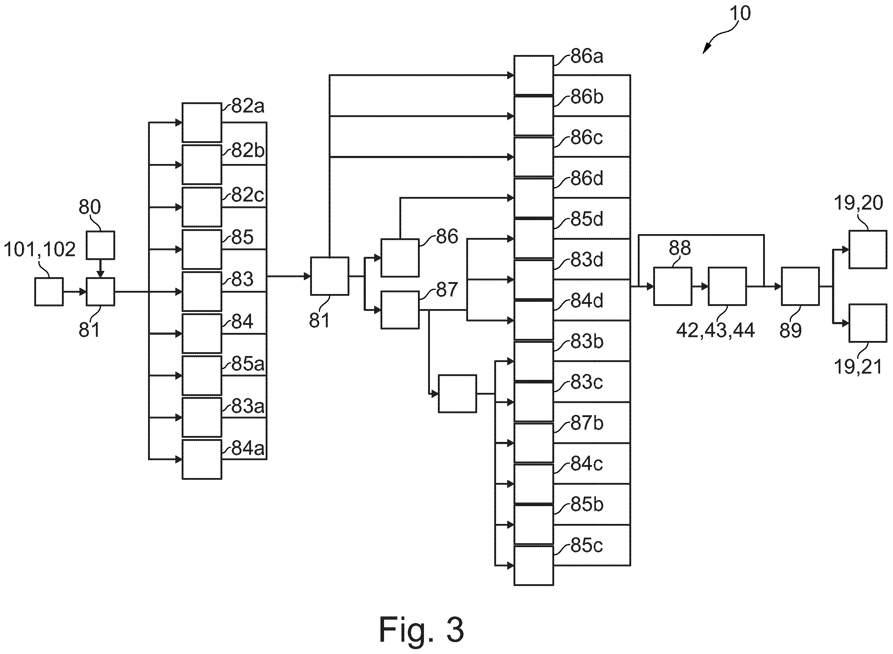

Figure 3 shows a method 10 for assessing a walking pattern. Figure 3 can be based on the method of figure 1 or 2 but shows more steps in some regard than figure 1 or 2. A sensor 101, namely an inertial measurement unit 102, is placed on the pelvis. As a next step, an offset calibration 80 of the measurement data can take place. Furthermore, a data interpolation 81 can take place, for example the data can be interpolated to 100 Hertz.

As a result, there is acceleration data 82a, 82b, 82c regarding different axis. Furthermore, a yaw 85, a pitch 83 and a roll 84 are determined as well as respective offsets, namely a pitch-offset 83a, a yaw-offset 85a and a roll-offset 84a. Another interpolation to a specific frequence, such as for example 100 Hertz, can take place 81. Linear accelerations can be determined 86. Different accelerations 86a to 86d can be determined. Furthermore, detrends can be determined 87, namely a yaw detrend 85d, a pitch detrend 83d, a roll detrend 84d.

In addition, the first derivatives and the second derivatives can be determined, namely the first derivative 83b and the second derivate 83c of the pitch, the first derivate 84b and the second derivative 84c of the roll and the first derivative 85b and the second derivative 85c of the yaw. The data can then be normalized 88 and a time analysis 42, frequency analysis 43 and/or time frequency analysis 44 can take place. A neural network used for assessing the beforementioned parameters can be deep learned 89 and a double support can be determined 90 as well as the left stances can be determined 20. Furthermore, a double support 19 in the right stands 21 can be determined.

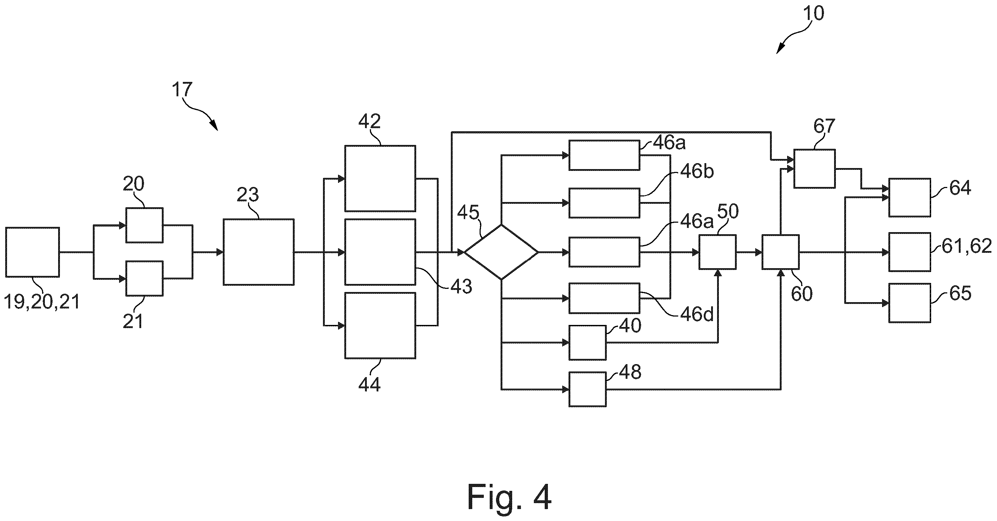

Figure 4 shows at least partly a method 10 for assessing a walking pattern of a person. The double support or in other words double stance 19, the rights stance 21 and the left stance 20 have been determined. The different phases of the walking pattern can be determined 23.

A time analysis 42, a frequency analysis 43 and/or a time frequency analysis 44 can take place. The signals can be processed 12a. The walking pattern can be assessed 17. By the different analysis possibilities, walking pattern features can be determined 45.

There can be a comparison 46 of the measurement data and/or the derivation data and/or the walking pattern features to previous data of the same person or to a predefined optimal walking pattern. For example, there can be a comparison 46a to a walking pattern which is to be changed, a comparison 46b to a recorded ideal walking pattern, a comparison 46c to an average of previous steps from the same side and/or a comparison 46 d to previous steps from the other side. In this way the asymmetry 40 can be detected as well as a walking disorder and/or walking deviation can be detected and indicated 48. A sensitivity 50 can be adapted and a feedback can be provided 60, especially, a live feedback is provided 61. Most preferred, vibration signals can be generated 62. Furthermore, a visual representation can be provided 64 and a traffic light system can be provided65. The tracking 67 of walking pattern improvements can take place.

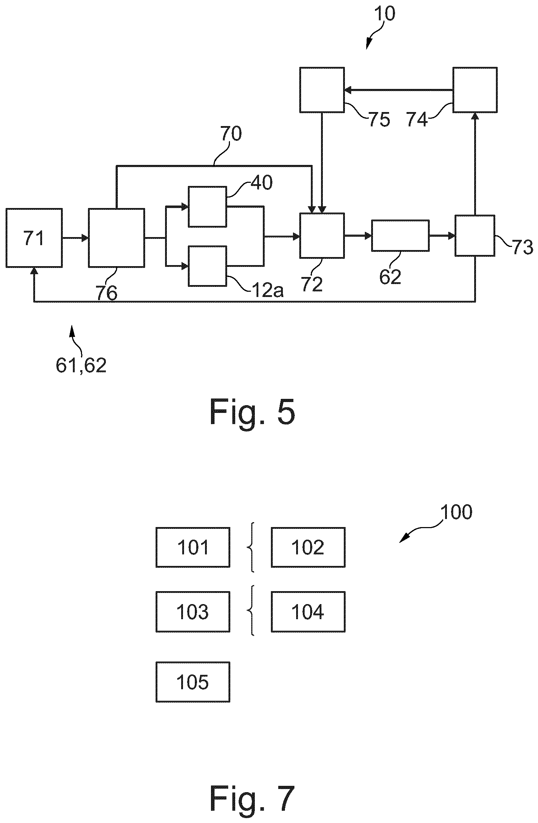

Figure 5 shows a part of a method 10 where a live feedback is provided, namely vibration signals are generated 62. First of all, there is a person's interpretation 71 of the vibration signal. A white box 76 follows as part of a control unit in which decisions regarding the feedback can take place. with an asymmetry detection 40 and a signal processing 12a of the measurement data and/or derivation data and/or the walking pattern features. Vibration signals can be adapted 70 depending on the activity. There can be a further signal processing 72 and a vibration signal 73 can be generated 62. There is an impedance coupling 74 between the walking pattern at the belt and the vibration signals can be measured 75.

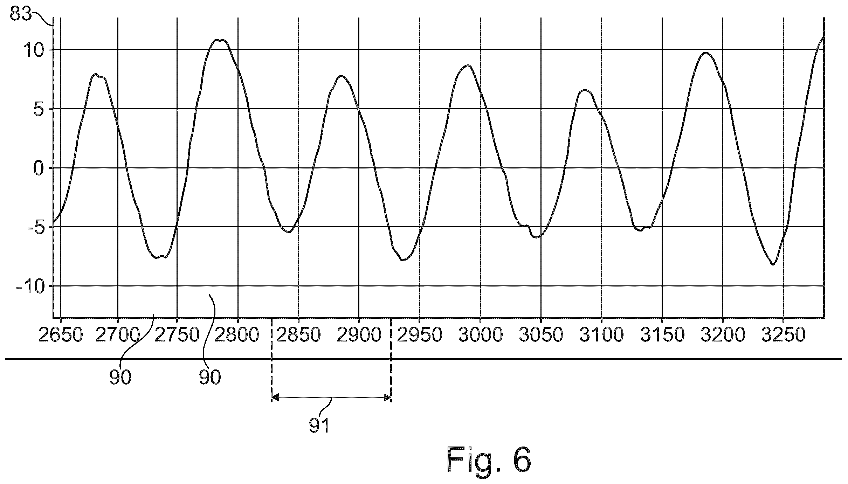

Figure 6 shows the yaw 85 over time. It can be seen that it follows a regular pattern from which conclusions on the walking pattern can be derived. The grey areas indicate the double stance 19. A walking pattern cycle 91 is indicated in the figure.

Figure 7 shows a device 100 processing a walking pattern of a person having a sensor 101, namely an inertial measurement unit 102, an evaluation unit 103, a neural network 104 and at least one feedback actuator 105.

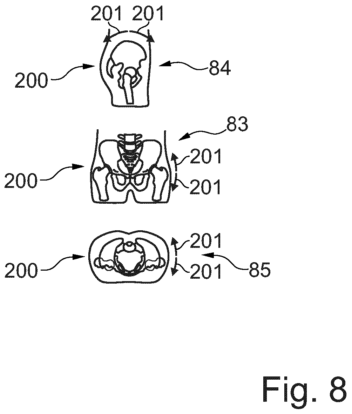

In figure 8, the pitch 83, roll 84 and yaw 85 at the pelvis 200 of a person is shown. In the upper figure, the roll 84 as the pelvic tilt is shown. A rotational movement 201 according to the tilt 84 can rotate the pelvis to the anterior or posterior side in a sagittal plane. In the middle figure, the pitch 83 as the pelvic obliquity is shown. A rotational movement 201 according to the pitch 83 can move the pelvis up (hike) or down (drop) at one side in the frontal plane. In the lower figure, the yaw 85 as the pelvic rotation is shown. A rotational movement 201 according to the yaw 85 can move the pelvis 200 backward or forward in a transverse plane.

### Reference signs

- 10: method for assessing a walking pattern of a person
- 11: activity detection
- 11a, 11b, 11c, 11d, 11e: activity types
- 12: pre-processing
- 12a: processing
- 13: measuring a roll, a pitch and a yaw of a walking movement of the person
- 14: measuring linear accelerations
- 15: determining the first derivates of the measurement data as derivation data
- 16: determining the second derivates of the measurement data as derivation data

- 17: assessing the walking pattern
- 18: evaluating the measurement data and the derivation data for assessing the walking pattern
- 19: determination of double support
- 20: recognition of left stance
- 21: recognition of right stance
- 22: preprocessing for each stance
- 23: determination of phases of the walking pattern
- 24: subdivision of the measurement data and the derivation data according to the phases of the walking pattern

- 40: determining an asymmetry of the walking pattern
- 42: time analysis of the measurement data and/or the derivation data
- 43: frequency analysis of the measurement data and/or the derivation data
- 44: time frequency analysis of the measurement data and/or the derivation data
- 45: determining walking pattern features
- 46: comparison of the measurement data and/or the derivation data and/or walking pattern features to previous data of the same person and/or to a pre-defined optimal walking pattern
- 46a: comparison to walking pattern which is to be changed
- 46b: comparison to recorded ideal walking pattern
- 46c: comparison to average of previous steps (same side)
- 46d: comparison with previous step (other side)
- 47: comparison of the measurement data and/or the derivation data and/or walking pattern features to predefined data regarding walking pattern disorders
- 48: indicating a walking disorder and/or deviation

- 50: adapt sensitivity

- 60: providing a feedback regarding the walking pattern to the person
- 61: providing a live feedback
- 62: generating vibration signals
- 63: generating different vibration signals regarding location and/or frequency and/or intensity
- 64: providing a visual representation of measurement data and/or walking pattern features
- 65: providing a traffic light system
- 66: determining a correct position and/or correct pressure of the sensor on the pelvis of the person by evaluating the vibration signals
- 67: tracking of walking pattern improvements

- 70: adapt vibration based on activity
- 71: user interpretation
- 72: signal processing
- 73: vibration signals
- 74: impedance coupling
- 75: measurement of vibration
- 76: white box

- 80: offset calibration
- 81: data interpolation
- 82a, 82b, 82c: acceleration data
- 83: pitch
- 83a: pitch offset
- 83b: 1st derivative of pitch
- 83c: 2nd derivative of pitch
- 83d: pitch detrend
- 84: roll
- 84a: roll offset
- 84b: 1st derivative of roll
- 84c: 2nd derivative of roll
- 84d: roll detrend
- 85: yaw
- 85a: yaw offset
- 85b: 1st derivative of yaw
- 85c: 2nd derivative of yaw
- 85d: yaw detrend
- 86: determining linear accelerations
- 86a, b, c, d: accerelations
- 87: determining detrend
- 88: data normalization
- 89: deep learning of neural network
- 90: double stance
- 91: walking pattern cycle

- 100: device for assessing a walking pattern of a person
- 101: sensor
- 102: inertial measurement unit
- 103: evaluation unit
- 104: neural network
- 105: feedback actuator

- 200: pelvis
- 201: rotational movement

## Claims

1. A method (10) for assessing a walking pattern of a person, **characterized in that**
the method (10) comprises
measuring (13) a roll, a pitch and a yaw of a walking movement of the person as measurement data by means of at least one sensor (101) placed on a pelvis of the person,
determining (15) the first derivates of the measurement data as derivation data, and
evaluating (18) the measurement data and the derivation data for assessing (17) the walking pattern.

2. The method (10) according to claim 1,
wherein the derivation data includes the second derivatives of the measurement data.

3. The method (10) according to claim 1 or 2,
wherein the measurement data includes linear accelerations.

4. The method (10) according to any one of the previous claims,
wherein the method (10) includes a determination (23) of phases of the walking pattern and a subdivision (24) of the measurement data and the derivation data according to the phases of the walking pattern.

5. The method (10) according to any one of the previous claims,
wherein the method (10) comprises determining (40) an asymmetry of the walking pattern regarding a left and a right side of the person.

6. The method (10) according to claim 5,
wherein the determination (40) of the asymmetry concerns a time analysis (42) and/or a frequency analysis (43) and/or a time frequency analysis (44) of the measurement data and/or the derivation data for determining (45) walking pattern features.

7. The method (10) according to claim 5 or 6,
wherein the determination (40) of the asymmetry comprises a comparison (46) of the measurement data and/or the derivation data and/or walking pattern features to previous data of the same person and/or to a pre-defined optimal walking pattern.

8. The method (10) according to any one of claims 5 to 7,
wherein the method (10) comprises a comparison (47) of the measurement data and/or the derivation data and/or walking pattern features to predefined data regarding walking pattern disorders, wherein based on the comparison (47) the method (10) comprises providing an indication (48) of a walking pattern disorder and/or deviation.

9. The method (10) according to any one of the previous claims,
wherein the method (10) comprises providing (60) a feedback regarding the walking pattern to the person.

10. The method (10) according to claim 9,
wherein
the feedback is provided (61) live and/or is provided (62) by vibration signals.

11. The method (10) according to claim 10,
wherein the method (10) comprises providing different vibration signals regarding location and/or frequency and/or intensity.

12. The method (10) according to claim 10 or 11,
wherein the method comprises determining (66) a correct position and/or correct pressure of the sensor on the pelvis of the person by evaluating the vibration signals.

13. A device (100) for assessing a walking pattern of a person, **characterized in that**
the device (100) includes at least one sensor (101) placed on a pelvis of the person for measuring the pitch, roll and yaw of a walking movement of the person as measurement data, and
an evaluation unit (103) for determining the first derivates of the measurement data,
and evaluating the measurement data and the derivation data for analyzing the walking pattern.

14. The device (100) according to claim 13,
wherein the sensor (101) includes an inertial measurement unit (102).

15. The device (100) according to claim 13,
wherein the device (100) includes at least one feedback actuator (105) to provide a feedback regarding the walking pattern to the person.
